# EUROPEAN PATENT APPLICATION

(11) **EP 3 392 243 A1**
(43) Date of publication of application: **24.10.2018**
(21) Application number: 15910639.2
(22) Date of filing: 16.12.2015
(51) Int. Cl.: C07D 311/16, C08G 18/32, C08L 75/04, C08L 75/08

(54) **SELF-REPAIRING POLYMERS**

(71) Applicant: Fundación Gaiker, 48170 Zamudio, Bizkaia (ES); Consejo Superior De Investigaciones Científicas, 28006 Madrid (ES)
(72) Inventor: SEOANE RIVERO, Rubén, E-48170 Zamudio - Bizkaia (ES); GONDRA ZUBIETA, Joseba Koldo, E-48170 Zamudio - Bizkaia (ES); BILBAO SOLAGUREN, Maria Pilar, E-48170 Zamudio - Bizkaia (ES); MARCOS FERNÁNDEZ, Ángel Antonio, E-28006 Madrid (ES); ARETXEDERRETA MONTERO, Unai, E-48300 Gernika - Bizkaia (ES)
(74) Representative: Balder IP Law, S.L.
(86) International application number: PCT/ES2015/070920
(87) International publication number: WO 2017/103295

(57) **Abstract**

The present invention relates to coumarins of formula (I), polyols and polyurethanes produced from said coumarin, as well as the method for producing said polyurethanes, the use thereof in the preparation of coating films and method thereof for repairing a damaged surface of said polyurethane. Formula (I)

## Description

### Field of the invention

The present invention relates to coumarins of formula (I), polyols and polyurethanes produced from said coumarin, as well as the method for producing said polyurethanes, the use thereof in the preparation of coating films and method thereof for repairing a damaged surface of said polyurethane.

### Background of the invention

Self-repairing systems of polymeric materials that are based on the microencapsulation of a repair agent, application of an external stimulus, such as heat or light, and supramolecular chemistry, such as H bonding, are known in the state of the art.

Microencapsulation is a system that has a high recovery percentage of the damaged polymer. However, it only enables the recovery to be carried out once, that is, if the same area is damaged again recovery is not possible. Other drawbacks of this system are the difficulty in ensuring that the contents of the microcapsules is capable of fully emerging to the exterior, the requirement of a catalyst in the polymer matrix, the high cost, the environmental toxicity, the stability and the processing of this type of material.

Self-repair using supramolecular chemistry entails the drawback that it does not enable transparent polymers to be produced, transparency being an essential aspect in many applications of polymers.

Self-repair by means of applying an external stimulus, such as light (photochemical self-repair), does not use catalysts. Therefore, it is a cost-effective system and does not harm the environment. Moreover, it enables transparent polymers to be produced.

Various photochemical polymer self-repair systems based on reversible photo-cross-linking reactions of chromophore groups have been described [Liu Y.-L. and Chuo T.-W., Polym Chem (2013), 4, 2194-2205; Froimowicz H. et al., Macromol Rapid Commun (2011), 32, 468-473; and Ghosh B. and Urban M.W., Science (2009), 323, 1458-1460]. Among the chromophore groups, the use of coumarins has been described due to their capacity to undergo a reversible dimerization [Ling J. et al., J Mater Chem (2011), 21, 18373-18380; Ling J. et al., Polymer (2012), 53, 2691-2698; and CN1021535856]. Said reversible dimerization takes place at wavelengths of about 350 nm or solar radiation, causing photodimerization [2+2] between two coumarins present in the polymeric structure giving rise to the formation of a cyclobutane ring, and the reverse reaction takes place at wavelengths lower than 260 nm, that is, photocleavage, and therefore, the two double bonds are regenerated again, giving rise to the initial coumarin, as shown in Scheme 1, wherein R represents the polymeric chain. This photodimerization-photocleavage enables the self-repair of the polymer in the damaged area since when the polymeric surface is damaged by mechanical stress, the weakest chemical bonds are those of the dimer, and therefore, these are the bonds that are cleaved. Upon being irradiated with light with a wavelength lower than 260 nm, the previously cleaved bonds of the coumarins dimerize and give rise to the polymer repair.

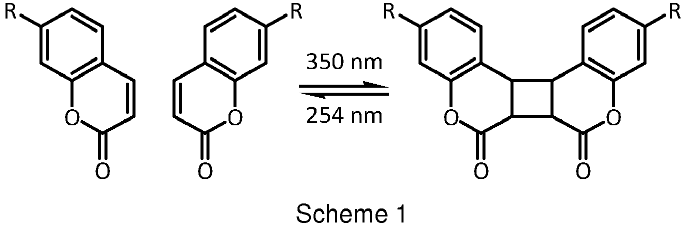

The reversibility of the photodimerisation-photocleavage reactions of the coumarins enables polymeric systems with multiple recovery to be produced, that is, the self-repair can take place as many times as necessary.

In particular, Ling et al. describes [J Mater Chem (2011), 21, 18373-18380] the self-repair of polyurethanes by introducing a phenolic coumarin derivative as a lateral chain of the structure of a polyurethane produced from a hexamethylene diisocyanate trimer, polyethylene glycol 400 and 7-hydroxyethoxy-4-methylcoumarin, the structure of which is shown below. However, the produced polyurethane has gelification problems, and therefore, it is difficult to apply it as a coating in the form of a film.

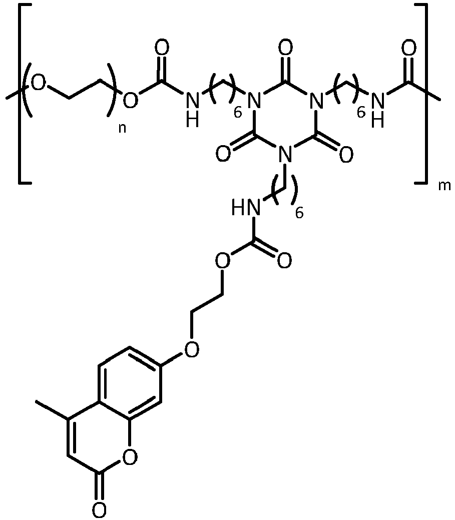

In order to resolve the gelification problems, Ling et al. [Polym (2012), 53, 2691-2698] describes the use of dihydroxylated coumarins in the synthesis of self-repairing polyurethanes, such that the coumarin is integrated in the main chain of the polyurethane. It specifically describes a polyurethane produced from isophorone isocyanate, polyethylene glycol 400 or 800 and 5,7-bis(2-hydroxyethoxy)-4-methylcoumarin, the structure of which is shown below.

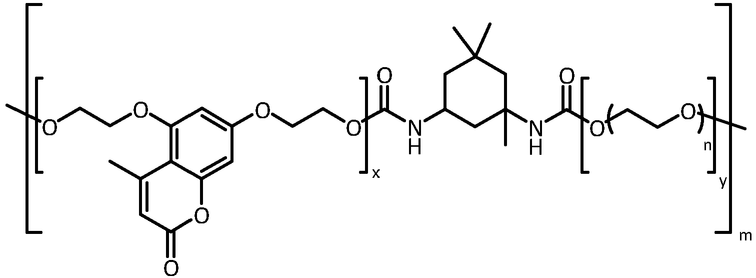

However, there is a need to have improved self-repairing polyurethanes with regards to mechanical properties, as well as the irradiation times required for the self-repair, and the efficiency of said self-repair.

Document ES 2537618 A1 describes self-repairing polyurethanes comprising dihydroxylated coumarin derivatives, the structure of which is shown below.

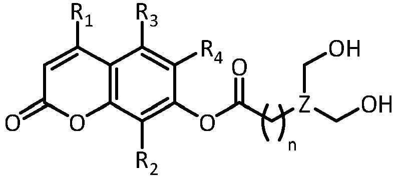

Polyurethane coatings comprising said dihydroxylated coumarin derivatives, which function as chain extenders, are highly reactive when irradiated, and therefore, have a high self-repair percentage, in addition to having the capacity to undergo multiple self-repair cycles, being transparent and having good mechanical performance. With regards to producing polyols from said derivative, as a consequence of the generated trans-esterification reaction, coumarin molecules not bonded to the polyol chain and species with functionality 3 which ultimately led to a cross-linked polyurethane were obtained.

Therefore, there is a need to have new coumarin derivatives, which in addition to acting as chain extenders generating photosensitive and self-repairing polyurethanes with good mechanical properties, particularly higher than those of a conventional coating (54 MPa of tensile strength) and capacity to undergo multiple self-repair cycles, enable polyols to be produced, that is, the coumarin derivative can act as a polyol initiator, preventing the transesterification reaction that was created when producing polyols with the previous coumarin derivative. In this case, photosensitive and self-repairing high-tenacity elastomers are created. There is also a need to have new polyol initiators, giving rise to photosensitive and self-repairing high-tenacity elastomers.

### Summary of the invention

Surprisingly, the inventors have discovered that polyurethanes formulated with coumarin of formula (I) according to the present invention as chain extenders give rise to photosensitive and self-repairing coatings that have the capacity to undergo multiple self-repair cycles, in addition to having good mechanical properties, particularly superior to those of a conventional coating. Said compounds of formula (I) can also act as polyol initiators, giving rise to high-tenacity elastomers.

In a first aspect, the invention relates to a compound of formula (I): 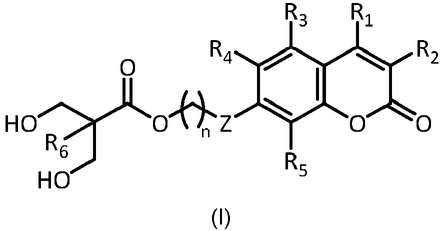
wherein R₁, R₂, R₃, R₄, R₅ and R₆ are independently selected from the group consisting of H, C₁-C₆ alkyl and C₁-C₆ alkoxyl;
Z is CH₂ or O; and n is a number selected from the group consisting of 1, 2, 3 and 4;
or a stereoisomer thereof.

In a second aspect, the invention relates to a polyol (A) obtainable by reacting one or more compounds of formula (I) as defined in the first aspect, with one or more compounds (B) in the presence of a catalyst, wherein compound (B) comprises:
a) a functional group selected from -C(=O)-O- and -O-, and optionally at least one hydroxyl group, provided that when the functional group selected from -C(=O)-O- and -O- does not form part of a cycle, the hydroxyl groups must be present; or
b) a functional group of the type -O-CH(CH₃)--(C=O)- and at least one hydroxyl group.

In a third aspect, the invention relates to a method for producing a polyurethane that comprises reacting a mixture comprising:
- one or more polyisocyanates (C) comprising at least two isocyanate groups,
- one or more polyols selected from the group consisting of a polyol (A) as defined in the second aspect and a compound of formula (I) as defined in the first aspect, or mixtures thereof, and
- optionally one or more polyols (D) with at least two hydroxyl groups selected from the group consisting of polyester polyols, polyether polyols, aliphatic polyols, polycarbonate polyols, polyether carbonate polyols and polyols of natural origin, in an aprotic organic solvent, in the presence of a catalyst,
wherein the ratio of equivalents of polyisocyanate (C) to the sum of equivalents of polyol (A), compound of formula (I) and polyol (D) in the reaction mixture is comprised between 2:1 and 1:1.2, and
wherein the ratio between the sum of the equivalents of polyol (A) and the equivalents of compound of formula (I) with respect to the equivalents of polyisocyanate (C) is comprised between 1% and 95%.

In a fourth aspect, the invention relates to a method for producing a polyurethane that comprises reacting a mixture comprising:
- one or more polyisocyanates (C) comprising at least two isocyanate groups,
- one or more polyols (D) with at least two hydroxyl groups selected from the group consisting of polyester polyols, polyether polyols, aliphatic polyols, polycarbonate polyols, polyether carbonate polyols and polyols of natural origin, and
- a compound of formula (I) as defined in the first aspect.
in an aprotic organic solvent,
in the presence of a catalyst,
wherein the ratio of equivalents of polyisocyanate (C) to the sum of equivalents of polyol (D) and compound of formula (I) in the reaction mixture is comprised between 2:1 and 1:1.2, and
wherein the ratio between the sum of the equivalents of polyol (D) and the equivalents of compound of formula (I) with respect to the equivalents of polyisocyanate (C) is comprised between 1% and 95%.

In a fifth aspect, the invention relates to a polyurethane obtainable by means of the method defined in the third and fourth aspects.

In a sixth aspect, the invention relates to the use of a polyurethane as defined in the fifth aspect in the preparation of a coating.

In a seventh aspect, the invention relates to a method for repairing a coating as defined in the sixth aspect that comprises exposing said coating to light comprising a radiation with a wavelength comprised between 310 nm and 370 nm.

### Description of the figures

Figure 1 shows the decrease in absorbance when the polyurethane of example 3 is irradiated with a wavelength of 350 nm.
Figure 2 shows the increase in absorbance when the polyurethane of example 3 is irradiated with a wavelength of 254 nm.
Figure 3 shows the self-repair capacity of the polyurethane of example 3 after damaging said polyurethane.
Figure 4 shows the self-repair capacity of the polyurethane of example 3 after 1 and 2 cycles of damage to said polyurethane.

### Detailed description of the invention

Within the context of the present invention, the term "alkyl" relates to a radical with a linear or branched hydrocarbon chain consisting of carbon and hydrogen atoms, without unsaturations, having 1 to 6, preferably 1 to 3 carbon atoms, and that is bonded to the rest of the molecule by means of a single bond, for example, methyl, ethyl, n-propyl, i-propyl, n-butyl, t-butyl, n-pentyl, etc.

The term "alkoxyl" as used herein relates to an alkyl radical, as defined above, bonded to the rest of the molecule by means of a -O- group, for example, methoxy, ethoxy, n-propoxy, isopropoxy, n-butoxy, t-butoxy.

The term "alkylene" as used herein relates to a radical with a linear hydrocarbon chain consisting of carbon and hydrogen atoms, which has the number of carbon atoms indicated in each case and that is bonded at both ends to the rest of the molecule by means of single bonds, for example, ethylene (-CH2-CH2-), n-propylene (-CH2-CH2-CH2-), n-butylene (-CH2-CH2-CH2-CH2-), n-pentylene (-CH2-CH2-CH2-CH2-CH2-), etc.

The term "cycloalkyl" as used herein relates to a saturated carbocyclic ring that has 3 to 8 carbon atoms, preferably 4 to 6 carbon atoms, for example, cyclopropyl, cyclobutyl, cyclopentyl, cyclohexyl, cycloheptyl and cyclooctyl.

The term "cycloalkylene" as used herein relates to a saturated carbocyclic ring that has 3 to 8 carbon atoms, preferably 4 to 6 carbon atoms, and that is bonded to the rest of the molecule through two different carbon atoms by means of single bonds, for example, cyclopropylene, cyclobutylene, cyclopentylene, cyclohexylene, cycloheptylene and cyclooctylene.

The term "aryl" as used herein relates to an aromatic hydrocarbon radical such as phenyl, naphthyl or anthracyl. The aryl radical may be optionally substituted by one or more substituents such as hydroxyl, halogen, alkyl and alkoxyl, as defined herein.

The term "halogen" or "halo", as used herein relates to -F, -CI, -Br and -I.

The term "hydroxyl" or "hydroxy" relates to an -OH group.

The term "aliphatic" as used herein relates to cyclic or acyclic, linear or branched, saturated or unsaturated hydrocarbon compounds, excluding aromatic compounds.

The term "aromatic" as used herein relates to a mono- or polycyclic hydrocarbon comprising at least one unsaturated ring that meets Hückel's rule of aromaticity. Examples of aromatic rings are phenyl, indanyl, indenyl, naphthyl, phenenthryl and anthracyl.

The term "stereoisomer" as used herein relates to compounds formed by the same atoms bonded by the same bond sequence but that have different three-dimensional structures that are not interchangeable, for example, isomers due to the presence of chiral centers (enantiomers, diastereomers and mixtures thereof including racemic mixtures) and isomers due to the presence of multiple bonds (*cis, trans* and mixtures thereof).

The term "equivalent", as used herein relates to the moles of compound per reactive units present in said compound. For example, one mole of diisocyanate equals to two equivalents of diisocyanate, whereas one mole of monoisocyanate equals to one equivalent of monoisocyanate.

### Compound of formula (I)

In the first aspect, the invention relates to a coumarin derivative that is a compound of formula (I), as defined above.

In a particular embodiment, the invention is directed to a compound of formula (I), as defined above, wherein R₁, R₂, R₃, R₄, R₅ and R₆ are independently selected from the group consisting of H, C₁-C₃ alkyl and C₁-C₃ alkoxyl; Z is CH₂ or O; and n is a number selected from the group consisting of 1, 2, 3 and 4.

In another particular embodiment, the invention is directed to a compound of formula (I), as defined above, wherein R₁ is selected from the group consisting of H, C₁-C₃ alkyl and C₁-C₃ alkoxyl; preferably from the group consisting of H, C₁-C₃ alkyl; even more preferably from the group consisting of H, methyl and ethyl; most preferably, R₁ is methyl.

In another particular embodiment, the invention is directed to a compound of formula (I), as defined above, wherein R₂ is selected from the group consisting of H, C₁-C₃ alkyl and C₁-C₃ alkoxyl; preferably from the group consisting of H, C₁-C₃ alkyl; even more preferably from the group consisting of H, methyl and ethyl; most preferably, R₂ is H.

In another particular embodiment, the invention is directed to a compound of formula (I), as defined above, wherein R₃ is selected from the group consisting of H, C₁-C₃ alkyl and C₁-C₃ alkoxyl; preferably from the group consisting of H, C₁-C₃ alkyl; even more preferably from the group consisting of H, methyl and ethyl; most preferably, R₃ is H.

In another particular embodiment, the invention is directed to a compound of formula (I), as defined above, wherein R₄ is selected from the group consisting of H, C₁-C₃ alkyl and C₁-C₃ alkoxyl; preferably from the group consisting of H, C₁-C₃ alkyl; even more preferably from the group consisting of H, methyl and ethyl; most preferably, R₄ is H.

In another particular embodiment, the invention is directed to a compound of formula (I), as defined above, wherein R₅ is selected from the group consisting of H, C₁-C₃ alkyl and C₁-C₃ alkoxyl; preferably from the group consisting of H, C₁-C₃ alkyl; even more preferably from the group consisting of H, methyl and ethyl; most preferably, R₅ is H.

In another particular embodiment, the invention is directed to a compound of formula (I), as defined above, wherein R₆ is selected from the group consisting of H, C₁-C₃ alkyl and C₁-C₃ alkoxyl; preferably from the group consisting of H, C₁-C₃ alkyl; even more preferably from the group consisting of H, methyl and ethyl; most preferably, R₆ is methyl.

In another particular embodiment, the invention is directed to a compound of formula (I), as defined above, wherein Z is CH₂.

In another particular embodiment, the invention is directed to a compound of formula (I), as defined above, wherein Z is O.

In another particular embodiment, the invention is directed to a compound of formula (I), as defined above, wherein n is a number selected from the group consisting of 1, 2 and 3; preferably from the group consisting of 1 and 2; most preferably, n is 2.

In a preferred embodiment, the present invention is directed to a compound of formula (I) wherein Z is O and n is selected from the group consisting of 1 and 2.

In another preferred embodiment, the present invention is directed to a compound of formula (I), as described above, wherein R₁ and R₆ are independently selected from the group consisting of H, methyl and ethyl, and R₂, R₃, R₄ and R₅ are H.

In another preferred embodiment, the present invention is directed to a compound of formula (I), as described above, wherein R₁ and R₆ are methyl, R₂, R₃, R₄ and R₅ are H, and Z is O.

In another preferred embodiment, the present invention is directed to a compound of formula (I), as described above, wherein R₁ and R₆ are methyl, R₂, R₃, R₄ and R₅ are H, Z is O and n is 2.

The compound of formula (I) can be obtained by an esterification reaction between the acid (IV) or a precursor thereof, such as an anhydride or acid halide and the corresponding coumarin (III), as shown in Scheme 2, by methods known to those skilled in the art and described in Smith M.B. and March J. in March's Advanced Organic Chemistry Reactions, Mechanisms, and Structure, 6th Ed. John Wiley & Sons.

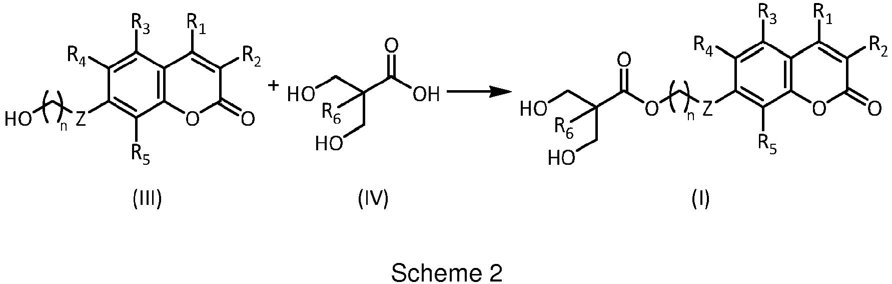

The alcohol groups of the acid (IV) can be protected by alcohol protective groups (PG) known to those skilled in the art and described, for example, in P.G.M. and Greene T.W. in Protecting groups in Organic Synthesis, 4th Ed. Wiley-Interscience, and in Kocienski P.J. in Protecting Groups, 3rd Ed. Georg Thieme Verlag, as shown in Scheme 3. For example, ethers (including the formation of acetal) and silylated derivatives.

The esterification reaction can be carried out starting with the acid (IV), preferably with its hydroxyl groups protected, by acid catalysis using methods known to those skilled in the art or it can also be carried out by activating the acid (IV), preferably with its hydroxyl groups protected, by forming the anhydride, for example in the presence of dimethylaminopyridine and subsequent reaction with coumarin (III).

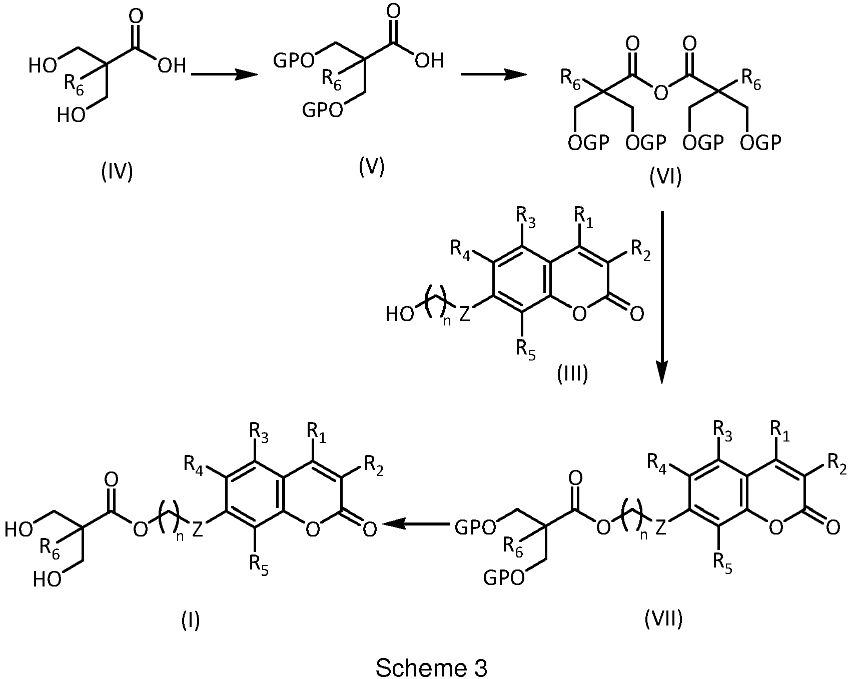

Preferably, the method for obtaining the compound of formula (I) comprises the steps defined in Scheme 3, that is, protection of the hydroxyl groups of the acid (IV) to yield the acid (V) using standard methods, followed by activation of the acid (V) by forming the anhydride (VI) using standard methods, such as, for example, in the presence of dicyclohexylcarbodiimide (DDC) and reacting the anhydride (VI) with the coumarin (III) to yield the ester (VII) which, after deprotecting the hydroxyl groups using standard methods, yields the compound of formula (I).

### Polyol (A)

In the second aspect, the invention relates to a polyol (A) obtainable by a condensation reaction of one or more compounds of formula (I), as defined above, with one or more compounds (B) in the presence of a catalyst, wherein compound (B) comprises:
a) a functional group selected from -C(=O)-O- and -O- (which is involved in the condensation reaction to bond the hydroxyl groups of the compound (I)), and optionally at least one hydroxyl group, provided that when the functional group selected from -C(=O)-O- and -O- does not form part of a cycle, the hydroxyl group must be present; or
b) a -O-CH(CH₃)-(C=O)- functional group and at least one hydroxyl group. In an embodiment of the present invention, the compound (B) is a
lactone or a cyclic ether, that is, an aliphatic heterocyclic compound comprising one or more -C(=O)-O- or -O- groups forming part of a cycle. Examples of this type of compound are lactones such as ε-caprolactone, butyrolactone, valerolactone, lactide, etc. and the copolymers between them; and cyclic ethers such ethylene oxide and propylene oxide.

In another embodiment of the present invention, the compound (B) comprises a -C(=O)-O- group or a -O- group (which will be involved in the reaction to bind to one of the hydroxyl groups of the compound (I)), and a hydroxyl group. Examples of this type of compound are hydroxy acids, which comprise a carboxylic acid at one end and a hydroxyl group at the other end, and hydroxy esters, which comprise an ester at one end and a hydroxyl group at the other end.

In another embodiment of the invention, the compound (B) comprises the -O-CH(CH₃)-(C=O)- functional group present in the lactic acid and at least one hydroxyl group. Examples of this type of compound are L-lactic and D-lactic acid and the oligomers thereof from L-lactic and D-lactic acid or mixtures thereof.

In a preferred embodiment of the invention, the compound (B) is a lactone; preferably selected from the group consisting of ε-caprolactone, δ-valerolactone, γ-butyrolactone, and β-propiolactone; more preferably selected from the group consisting of ε-caprolactone and δ-valerolactone; most preferably, the lactone is ε-caprolactone.

The catalyst used can be a tertiary amine such as, for example, triethylamine, triethylenediamine, methylethanolamine, triethanolamine, dimethylethanolamine, pyridine, 1,4-diazabicyclo[2.2.2]octane, dimethylcyclohexylamine and dimethylpiperazine; organic derivatives of tin, mercury, lead, bismuth, zinc and potassium such as, for example, tin 2-ethylhexanoate (commonly referred to as tin octanoate), tin isooctanoate, dibutyltin dilaurate, potassium octanoate and potassium acetate. Preferably, the catalyst is selected from the group consisting of tin octanoate, tin isooctanoate, dibutyltin dilaurate, triethylamine, triethylenediamine, methylethanolamine, triethanolamine, dimethylethanolamine, pyridine, 1,4-diazabicyclo[2.2.2]octane and dimethylcyclohexylamine; more preferably it is independently selected from the group consisting of tin octanoate, tin isooctanoate and dibutyltin dilaurate; most preferably, the catalyst is tin octanoate.

In a particular embodiment, the reaction is carried out in the absence of solvent.

In another embodiment, the reaction is carried out in the presence of an aprotic organic solvent independently selected from the group consisting of dimethylformamide, butyl acetate, dimethyl sulfoxide, dimethylacetamide, tetrahydrofuran, dioxane, ethyl acetate, acetone, cyclohexanone, methyl ethyl ketone, acetonitrile, hexane, toluene, dichloromethane and mixtures thereof; more preferably, it is independently selected from the group consisting of dimethylformamide, butyl acetate, dimethylacetamide, dimethyl sulfoxide and mixtures thereof; most preferably, the aprotic organic solvent is dimethylformamide, butyl acetate, or mixtures thereof.

Preferably, the reaction is carried out at a temperature comprised between 50 °C and 150 °C, more preferably between 70°C and 130 °C, most preferably between 90 °C and 110 °C.

In a particular embodiment, the invention is directed to a polyol (A) obtainable by reacting one or more compounds (B) and one or more compounds of formula (I), wherein the molar ratio of compounds (B) to compounds of formula (I) is comprised between 80:1 and 1:1, more preferably between 40:1 and 1.5:1.

In a particular embodiment, the invention is directed to a polyol (A) having an average molecular weight from 200 Dalton to 10,000 Dalton, preferably from 200 Dalton to 2000 Dalton.

In another preferred embodiment, the polyol (A) is a compound of formula (II): 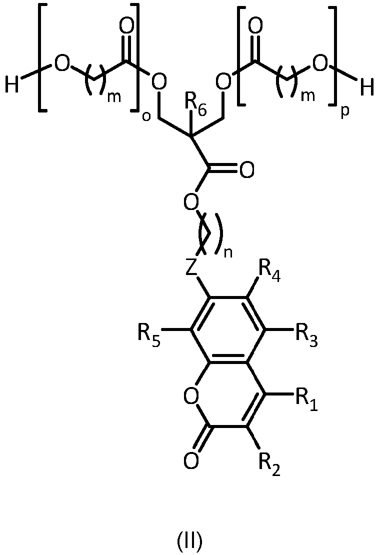 wherein R₁, R₂, R₃, R₄, R₅, R₆, Z and n are as defined for the compound of formula (I), and o and p are independently selected from a number comprised between 0 and 40, provided that at least one of o and p is not zero.

### Polyurethane

In general, the polyurethanes are produced from three monomers: a relatively long-chain flexible polyol, which forms the soft segments of the polyurethane, a polyisocyanate, and a short-chain polyol, which is also referred to as a chain extender if it is bifunctional or cross-linker if it has a functionality greater than 2. The reaction of the polyisocyanate with the chain extender forms the hard segments of the polyurethane.

In the third aspect, the present invention relates to a method for producing a polyurethane comprising the coumarin derivative of formula (I) as defined above, wherein said method comprises reacting a mixture comprising:
- one or more polyisocyanates (C) comprising at least two isocyanate groups,
- one or more polyols selected from the group consisting of a polyol (A) as defined above and a compound of formula (I) or mixtures thereof, and
- optionally one or more polyols (D) with at least two hydroxyl groups selected from the group consisting of polyester polyols, polyether polyols, aliphatic polyols, polycarbonate polyols, polyether carbonate polyols and polyols of natural origin,
in an aprotic organic solvent,
in the presence of a catalyst,
wherein the ratio of equivalents of polyisocyanate (C) to the sum of equivalents of polyol (A), compound of formula (I) and polyol (D) in the reaction mixture is comprised between 2:1 and 1:1.2, and
wherein the ratio between the sum of the equivalents of polyol (A) and the equivalents of compound of formula (I) with respect to the equivalents of polyisocyanate (C) is comprised between 1% and 95%, preferably between 2% and 60%.

In a particular embodiment, the method of the third aspect comprises reacting the mixture defined above, wherein both the polyol (A) and the compound of formula (I) are present. In this particular embodiment, the ratio of equivalents of polyisocyanate (C) to the sum of equivalents of polyol (A), compound of formula (I) and polyol (D) in the reaction mixture is preferably comprised between 2:1 and 1:1.2.

In another particular embodiment, the method of the third aspect comprises reacting the mixture defined above, wherein the polyols of the mixture do not comprise the polyol (A) (that is, the compound of formula (I) and the polyol (D) are present) or wherein the polyols of the mixture do not comprise the compound of formula (I) (that is, the polyol (A) and the polyol (D) are present). In these particular embodiments, the ratio of equivalents of polyisocyanate (C) to the sum of equivalents of either polyol (A) and polyol (D), or compound of formula (I) and polyol (D), in the reaction mixture is comprised between 2:1 and 1:1.2.

In a fourth aspect, the invention relates to a method for producing a polyurethane that comprises reacting a mixture comprising:
- one or more polyisocyanates (C) comprising at least two isocyanate groups,
- one or more polyols (D) with at least two hydroxyl groups selected from the group consisting of polyester polyols, polyether polyols, aliphatic polyols, polycarbonate polyols, polyether carbonate polyols and polyols of natural origin, and
- a compound of formula (I) (which would act as a chain extender).
in an aprotic organic solvent,
in the presence of a catalyst,
wherein the ratio of equivalents of polyisocyanate (C) to the sum of equivalents of polyol (D) and compound of formula (I) in the reaction mixture is comprised between 2:1 and 1:1.2, and
wherein the ratio between the sum of the equivalents of polyol (D) and the equivalents of compound of formula (I) with respect to the equivalents of polyisocyanate (C) is comprised between 1% and 95%.

The term "polyisocyanate", within the context of the present invention, should be construed as a compound comprising two or more, preferably two, aliphatic and/or aromatic, preferably aliphatic isocyanate groups. Examples of aliphatic polyisocyanates are hexamethylene diisocyanate (HDI), 2,2,4-trimethyl hexamethylene diisocyanate (TMDI), tetramethylene diisocyanate, octamethylene diisocyanate, decamethylene diisocyanate, dodecamethylene diisocyanate, tetradecamethylene diisocyanate, dimeryl diisocyanate (DDI), 1,1,6,6-tetrahydroperfluorohexamethylene diisocyanate (TFDI), isophorone diisocyanate (IPDI), 1,4-cyclohexane diisocyanate (CDI), 1,3-dicyclohexane diisocyanate, 1,2-dicyclohexane diisocyanate, 1,3-bis(isocyanatomethyl)cyclohexane (H₆XDI), 4,4'-dicyclohexylmethane diisocyanate (H₁₂MDI) and mixtures thereof. Examples of aromatic polyisocyanates are 2,4- and 2,6-toluene diisocyanate (TDI), para-phenylene diisocyanate (PPDI), 4,4'-diphenylmethane diisocyanate and the 2,4' and 2,2' isomers thereof (MDI), tetramethyl xylene diisocyanate (TMXDI), 1,5-naphthalene diisocyanate (NDI), 1,3-bis(1-isocyanate-1-methylethyl)benzene, meta-xylylene diisocyanate and mixtures thereof.

In a preferred embodiment, the polyisocyanate (C) is selected from the group consisting of linear or branched C₂-C₂₀-alkylene diisocyanate optionally substituted with 1 to 10 substituents independently selected from halogen, C₃-Cs-cycloalkylene diisocyanate optionally substituted with 1 to 4 substituents independently selected from the group consisting of C₁-C₃ alkyl and halogen, C₁-C₆-alkylen - C₃-C₈-cycloalkylene diisocyanate optionally substituted with 1 to 4 substituents independently selected from the group consisting of C₁-C₃ alkyl and halogen, and C₃-C₈-cycloalkylen - C₁-C₆-alkylen - C₃-C₈-cycloalkylene diisocyanate optionally substituted with 1 to 4 substituents independently selected from the group consisting of C₁-C₃ alkyl and halogen; preferably the polyisocyanate (C) is selected from the group consisting of hexamethylene diisocyanate (HDI), 2,2,4-trimethyl hexamethylene diisocyanate (TMDI), tetramethylene diisocyanate, octamethylene diisocyanate, decamethylene diisocyanate, dodecamethylene diisocyanate, tetradecamethylene diisocyanate, dimeryl diisocyanate (DDI), 1,1,6,6-tetrahydroperfluorohexamethylene diisocyanate (TFDI), isophorone diisocyanate (IPDI), 1,4-cyclohexane diisocyanate (CDI), 1,3-dicyclohexane diisocyanate, 1,2-dicyclohexane diisocyanate, 1,3-bis(isocyanatomethyl)cyclohexane (H₆XDI), 4,4'-dicyclohexylmethane diisocyanate (H₁₂MDI) and mixtures thereof; more preferably it is selected from the group consisting of hexamethylene diisocyanate (HDI), 2,2,4-trimethyl hexamethylene diisocyanate (TMDI), tetramethylene diisocyanate, octamethylene diisocyanate, decamethylene diisocyanate, dodecamethylene diisocyanate, tetradecamethylene diisocyanate; most preferably, the polyisocyanate is hexamethylene diisocyanate (HDI).

The polyol (D) with at least two hydroxyl groups relates to a compound that has at least two hydroxyl groups and is selected from the group consisting of polyester polyols, polyether polyols, aliphatic polyols (these also include polybutadiene polyols, wherein the aliphatic fragment is a chain from the polymerization of one or several butadiene groups), polycarbonate polyols, polyether carbonate polyol and polyols of natural origin as mentioned above. Preferably, the polyol (D) is selected from the group consisting of polyester polyols, polyether polyols, aliphatic polyols (these also include polybutadiene polyols, wherein the aliphatic fragment is a chain from the polymerization of one or several butadiene groups), polycarbonate polyols and polyether carbonate polyols. More preferably, it is selected from the group consisting of polyester polyols and polyether polyols.

Preferably, the polyols have an average molecular weight comprised between 200 Dalton and 10,000 Dalton, preferably between 200 Dalton and 4000 Dalton; more preferably, such polyols are selected from the group consisting of polyester polyols and polyether polyols.

The aliphatic polyols only comprise the terminal hydroxyl groups as functional groups. Preferred aliphatic polyols are diols such as 1,2-butanediol, 1,3-butanediol, 1,4-butanediol, 2,3-butanediol, 1,3-propanediol, 1,2-propanediol and mixtures thereof. The aliphatic polyols also include polybutadienes, wherein the aliphatic fragment is one or several butadiene groups.

The polyether polyols comprise, in addition to the terminal hydroxyl groups, non-terminal ether groups. The polyether polyols include polyethylene glycols, polypropylene glycol, mixed polyglycols based on ethylene oxide and propylene oxide, polytetramethylene glycols and polytetrahydrofurans.

The polyester polyols comprise, in addition to the terminal hydroxyl groups, non-terminal ester groups. The polyester polyols are produced by the condensation of diols and carboxylic acids, the anhydrides thereof and esters. Examples of polyester polyols are the condensation products based on ethylene glycol, 1,4-butylene glycol, 1,6-hexamethylene glycol or neopentyl glycol, with adipic acid or isophthalic acid. Polycaprolactones and (meth)acrylic polyols also belong to the group of polyester polyols. Polycaprolactones are obtained from the reaction between phosgene, aliphatic carbonates or aromatic carbonates, such as diphenyl carbonate or diethyl carbonate, with dihydric or polyhydric alcohols. Polycaprolactones are produced by the polyaddition of lactones such as, for example, ε-caprolactone, with an initiator compound having reactive hydrogen atoms, such as water, alcohols, amines or bisphenol A. (Meth)acrylic polyols are produced by radical copolymerization of (a) monomers of (meth)acrylic acids or esters, such as acrylic acid, methyl acrylate, ethyl acrylate, propyl acrylate, butyl acrylate and hexyl acrylate, methacrylic acid, methyl methacrylate, ethyl methacrylate, propyl methacrylate, butyl methacrylate and hexyl methacrylate and (b) monomers of hydroxyalkyl (meth)acrylates, such as hydroxyethyl acrylates, hydroxyethyl methacrylates, hydroxypropyl acrylates, hydroxypropyl methacrylates, glycidyl acrylate and glycidyl methacrylate.

The polycarbonate polyols comprise, in addition to the hydroxyl groups, non-terminal carbonate groups. Examples of these polyols are hexanediol polycarbonate, butanediol polycarbonate, 1,2-propanediol polycarbonate and other diols and mixtures of diols such as, for example, Oxymer from Perstorp, Eternacoll from UBE, and Desmophen from Bayer.

Combinations of polyester polyols, polycaprolactone polyols, (meth)acrylic polyols and polycarbonate polyols can also be used.

Polyether carbonate polyols comprise, in addition to the terminal hydroxyl groups, carbonate groups and ether groups. Examples of polyether carbonate polyols are the polyether carbonate polyols produced from propylene oxide and carbon anhydride (CO₂) with an alternating or random structure, with different carbonate group contents within the structure thereof such as, for example, Converge from Novomer.

The polyols of natural origin are those from vegetable oils. As examples, castor oil or the polyols produced by the derivatization of oils such as soybean, sunflower or canola oil may be mentioned.

In a preferred embodiment, the polyol (D) with at least two hydroxyl groups is selected from the group consisting of polycaprolactones, polyethylene glycols, polypropylene glycols, mixed ethylene oxide and propylene oxide polyglycols, polytetramethylene glycols, polytetrahydrofurans and mixtures thereof, wherein the polyol (D) has an average molecular weight between 200 Dalton and 10,000 Dalton, preferably between 200 Dalton and 10,000 Dalton; preferably the polyol (D) is selected from the group consisting of polycaprolactones, polyethylene glycols, polypropylene glycols, mixed ethylene oxide and propylene oxide polyglycols and mixtures thereof, wherein the polyol (D) has an average molecular weight between 200 Dalton and 10,000 Dalton; most preferably, the polyol (D) is selected from the group consisting of polycaprolactones, polyethylene glycols and mixtures thereof, wherein the polyol (D) has an average molecular weight between 200 Dalton and 4000 Dalton.

The aprotic organic solvent is preferably an organic solvent selected from the group consisting of dimethylformamide, butyl acetate, dimethyl sulfoxide, dimethylacetamide, tetrahydrofuran, dioxane, ethyl acetate, acetone, cyclohexanone, methyl ethyl ketone, acetonitrile, hexane, toluene, dichloromethane and mixtures thereof; preferably, it is selected from the group consisting of dimethylformamide, butyl acetate, dimethylacetamide, dimethyl sulfoxide and mixtures thereof; most preferably, the aprotic organic solvent is dimethylformamide, butyl acetate, or mixtures thereof.

The catalyst is a catalyst suitable for carrying out the reaction for forming the polyurethane, such as tertiary amines such as, for example, triethylamine, triethylenediamine, methylethanolamine, triethanolamine, dimethylethanolamine, pyridine, 1,4-diazabicyclo[2.2.2]octane, dimethylcyclohexylamine, dimethylpiperazine; organic derivatives of tin, mercury, lead, bismuth, zinc or potassium such as, for example, tin octanoate, tin isooctanoate, dibutyltin dilaurate, potassium octanoate and potassium acetate.

In a preferred embodiment, the catalyst is selected from the group consisting of tin octanoate, tin isooctanoate, dibutyltin dilaurate, triethylamine, triethylenediamine, methylethanolamine, triethanolamine, dimethylethanolamine, pyridine, 1,4-diazabicyclo[2.2.2]octane, dimethylcyclohexylamine and potassium octanoate; more preferably it is selected from the group consisting of tin octanoate, tin isooctanoate, dibutyltin dilaurate and potassium octanoate; most preferably, the catalyst is tin octanoate.

Typically, the catalyst is added in an amount comprised between 0.01% and 5% in moles with respect to the moles of polyisocyanate (C), preferably between 0.01% and 0.05% in moles with respect to the moles of polyisocyanate (C).

The method for forming polyurethane preferably is carried out at a temperature comprised between 50 °C and 150 °C, more preferably 50 °C and 100 °C, most preferably between 65 °C and 85 °C.

Typically, the formation of polyurethanes is carried out by stirring for a period of time no longer than 24 hours, preferably less than 12 hours, followed by heating at a temperature higher than room temperature, and no higher than 150°C, preferably lower than 100°C, most preferably between room temperature and 85 °C.

In a particular embodiment, the method according to the invention comprises reacting a mixture comprising one or more polyisocyanates (C) and one or more polyols (A).

In another particular embodiment, the method according to the invention comprises reacting a mixture comprising one or more polyisocyanates (C), one or more polyols (A) and one or more polyols (D).

In another particular embodiment, the method according to the invention comprises reacting a mixture comprising one or more polyisocyanates (C), one or more compounds of formula (I) and one or more polyols (D).

In another particular embodiment, the method according to the invention comprises reacting a mixture comprising one or more polyisocyanates (C), one or more compounds of formula (I) and one or more polyols (A).

In a preferred embodiment, the method according to the invention comprises reacting a mixture comprising one or more polyisocyanates (C), one or more compounds of formula (I), one or more polyols (A) and one or more polyols (D).

In an embodiment of the method of the invention, the ratio of equivalents of polyisocyanate (C) to the sum of equivalents of compound of formula (I), polyol (A) and polyol (D) in the reaction mixture is comprised between 2:1 and 1:1.2, preferably between 1.5:1 and 1:1.1, more preferably between 1.2:1 and 1:1.1, even more preferably 1.1:1 and 1:1.05, most preferably 1.05:1. In the method of the invention, the polyols present in the reaction mixture can be the compound of formula (I), polyol (A) and polyol (D), or it can be carried out in the absence of the compound of formula (I) (that is, the polyols present in the reaction mixture would be the polyol (A) and polyol (D)) or in the absence of the polyol (A) (that is, the polyols present in the reaction mixture would be the compound of formula (I) and the polyol (D)).

In another embodiment of the method of the invention, the ratio of equivalents of polyisocyanate (C) to the sum of equivalents of polyol (A) and polyol (D) in the reaction mixture is comprised between 2:1 and 1.2:1, preferably between 1.5:1 and 1.1:1, more preferably between 1.2:1 and 1.1:1, even more preferably 1.1:1 and 1.05:1, most preferably 1.05:1.

In an embodiment of the method of the invention, the ratio between the sum of the equivalents of polyol (A) and the equivalents of compound of formula (I) with respect to the equivalents of polyisocyanate (C) is comprised between 1% and 95%, preferably between 2% and 60%.

In a particular embodiment, the invention is directed to a method for producing polyurethanes as defined above comprising:
- reacting a mixture comprising a polyisocyanate (C) that comprises at least two isocyanate groups,
- a compound of formula (I) as defined above, and
- a compound of formula (II) (polyol (A)) as defined above,
in an aprotic organic solvent,
in the presence of a catalyst,
wherein the ratio of equivalents of polyisocyanate (C) to the equivalents of polyol (A) and compound of formula (I) in the reaction mixture is comprised between 2:1 and 1:1.2, preferably between 1.5:1 and 1:1.1, more preferably between 1.2:1 and 1:1.1, even more preferably between 1.1:1 and 1:1.05, most preferably 1.05:1, and wherein the ratio between the sum of the equivalents of polyol (A) and the equivalents of compound of formula (I) with respect to the equivalents of polyisocyanate (C) is comprised between 1% and 95%, preferably between 2% and 60%.

In a preferred embodiment, the invention is directed to a method for producing polyurethanes as defined above comprising:
- reacting a mixture comprising a polyisocyanate (C) selected from the group consisting of hexamethylene diisocyanate, isophorone diisocyanate, tetramethylene diisocyanate, octamethylene diisocyanate, decamethylene diisocyanate, dodecamethylene diisocyanate, and tetradecamethylene diisocyanate, preferably selected from the group consisting of hexamethylene diisocyanate and isophorone diisocyanate,
- a compound of formula (I) as defined above, and
- a compound of formula (II) (polyol (A)) as defined above, and
- optionally one or more polyols (D) with at least two hydroxyl groups selected from the group consisting of polyester polyols, polyether polyols, aliphatic polyols, polycarbonate polyols and polyether carbonate polyol, as defined above,
in an aprotic organic solvent,
in the presence of a catalyst,
wherein the ratio of equivalents of polyisocyanate (C) to the equivalents of polyol (A), compound of formula (I) and polyol (D) in the reaction mixture is comprised between 2:1 and 1:1.2, preferably between 1.5:1 and 1:1.1, more preferably between 1.2:1 and 1:1.1, even more preferably between 1.1:1 and 1:1.05, most preferably 1.05:1, and wherein the ratio between the sum of the equivalents of polyol (A) and the equivalents of compound of formula (I) with respect to the equivalents of polyisocyanate (C) is comprised between 1% and 95%, preferably between 2% and 60%.

In another aspect, the present invention relates to a polyurethane obtainable using the methods defined above.

### Uses of the polyurethane

Another aspect relates to the use of said polyurethane in the preparation of a coating, preferably incorporated as a varnish and paint additive, preferably in varnishes and paints for plastic and metal substrates, more preferably in varnishes and paints for the automotive industry.

The coating preferably has a thickness less than 150 µm, more preferably less than 140 µm, more preferably less than 130 µm, more preferably less than 120 µm, more preferably less than 110 µm, 100 µm, more preferably less than 90 µm, even more preferably less than 80 µm, even more preferably less than 70 µm, even more preferably less than 60 µm, even more preferably less than 50 µm, even more preferably less than 40 µm, even more preferably less than 30 µm, most preferably less than 20 µm.

The coating agents comprising the polyurethanes according to the invention are suitable for all fields of use wherein painting and coating systems are used, in particular in those with high quality requirements of the surface and the resistance of the films, e.g., coating of surfaces mineral construction materials, varnishing and sealing of wood and materials derived from wood, coating of metal surfaces (metal coating), coating and lacquering of asphalt or bituminous coatings, coating and lacquering of plastic surfaces (plastic coating) as well as high-gloss lacquers, in particular for the automotive industry.

The coating agents containing the polyurethanes according to the invention are commonly used in single-layer lacquers or in the transparent or cover layer (upper layer) of multi-layer structures.

The application of the coating can be carried out by different spraying methods such as, for example, compressed air, direct or electrostatic spraying using spraying facilities with one or, if necessary, two components. However, the lacquers and coating agents containing the above describe dispersions may be applied using different methods, such as by spreading, rollers or scrapers.

### Repair of the polyurethane

Said polyurethane (coating) defined above incorporates coumarin fragments of formula (I) in the structure thereof conferring the self-repairing properties by photodimerization-photocleavage of the coumarin derivatives, and provides for obtaining multiple-recovery polymeric systems.

Therefore, in another aspect, the present invention relates to a method for repairing a coating that comprises polyurethane as defined above comprising exposing said coating to light that comprises radiation with a wavelength comprised between 310 nm and 370 nm, preferably between 340 nm and 360 nm, preferably for 120 minutes to 180 minutes.

Said exposure to radiation comprising the defined wavelengths achieves the formation of dimers of the coumarin derivative and, therefore, the repair of the polyurethane.

Preferably, the exposure is carried out with light comprising radiation with wavelengths comprised between 330 nm and 370 nm; more preferably between 340 nm and 360 nm; most preferably at 350 µm.

"Repair of a coating" must be understood as the reduction of the number or magnitude of defects present in said coating, wherein "defects" are meant as perceptible discontinuities when examining the coating at a magnification of x20. Examples of said defects are scratches and cracks.

In a more preferred embodiment, the repairing method comprises a prior step of exposing a coating comprising the polyurethane to light comprising a radiation with a wavelength comprised between 200 nm and 260 nm, preferably for 1 minute to 20 mins.

Preferably, the irradiation is carried out at wavelengths comprised between 200 nm and 260 nm; more preferably between 240 nm and 260 nm; most preferably at 254 µm.

Said irradiation cleaves the dimers that were not cleaved when the polyurethane was damaged, and the dimers are subsequently formed again by the irradiation step defined above.

Preferably, the defects have a depth less than 70 µm, more preferably less than 65 µm, even more preferably less than 60 µm, even more preferably less than 55 µm, even more preferably less than 50 µm, even more preferably less than 45 µm, even more preferably less than 40 µm, even more preferably less than 35 µm, even more preferably less than 30 µm, even more preferably less than 25 µm, most preferably less than 20 µm.

The following examples are merely illustrative and must not be construed as limiting of the invention.

### Examples

### Materials and methods

IR spectra were recorded by a Perkin-Elmer Spectrum One FT-IR spectrometer equipped with an ATR (attenuated total reflectance spectrometer). In order to carry out the measurement, 4 scans at a resolution of 4 cm⁻¹ were carried out.

The 1H and 13C NMR spectra were recorded at room temperature in a Varian Inova 400 spectrometer (400 MHz 1H and 100 MHz 13C). DMSO-d6 was used as a solvent. The spectra were referenced with the residual signal of the solvent [δ (ppm) 2.50 (1H) and 39.51 (13C)].

The characterization of the thermal properties of the synthesized polyurethanes was carried out in a Mettler Toledo DSC822e calorimeter, recording the spectra at a heating rate of 10°C/min. In order to observe the photoreversibility of the cleaving-dimerization of the coumarin derivative comprised within the polyurethane coatings, UV-Visible spectroscopy (Perkin Elmer Lambda 35 spectrometer) and a Raman Renishaw inVia microscopic spectrophotometer were used. The film (coating) was made by evaporating a DMF solution on one face of a quartz cuvette. In the case of UV-Visible spectroscopy, it was recorded between 240 nm and 400 nm to quantify this photoreversibility.

The mechanical properties were tested in a MTS Synergie 200 testing machine equipped with a 100 N load cell. The samples tested were 3.8 mm wide and 30 mm long. The test was carried out at a rate of 5 mm·min⁻¹.

The damage of the coating is either carried out with the Erichsen 239-II scratcher, which allows for scratching with forces ranging from 1 to 20 N. This device has a tip with a diameter of 1 mm, which slides 2.2 cm over the coating at a speed of 0.02 m/s or by indentation. In this case, a tip with a diameter of 0.75 mm is used and a certain force is applied for a set time on the material. Lastly, the damage created on the materials is evaluated using confocal microscopy.

This study uses UV radiation to carry out the photodimerization and photocleavage reaction of the synthesized polyurethanes. It must be noted that two types of UV ovens were used:
- UVP 1000: irradiation at 254 nm and 365 nm
- UV DYMAX 2000-PC system: irradiation at 320-400 nm

To quantify the self-repair, the equipment used is a 3D optical profilometer from SENSOFAR (PLµ NEOX model), which allows measuring the roughness of the surface of the sample at micrometric and nanometric scale without the need of contact.

### Example 1: Synthesis of the 4-hydroxy-3-hydroximethyl-3-methylbutyric acid 4-metylcoumarin-7-ethoxyl ester

### Synthesis of 2,2,5-trimethyl-[1,3]dioxane-5-carboxylic acid (DMPA)

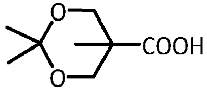

To a 500 mL round-bottom flask 50 g of 2,2-bis(methoxy)propionic (0.373 mol) (DMPA), 69 mL of 2,2-dimethoxypropane (0.569 mol) (DMP) and 3.55 g (0.0187 mol) of p-toluenesulphonic acid monohydrate were added. Subsequently, 250 mL of acetone (58.08 g/mol) were added and stirred at room temperature for 4 hrs. After this period of time, 18 mL of 2M NH₃ in EtOH (0.036 mol) are added. A fine white precipitate appears. The reaction is placed in a rotatory evaporator and the solvent is removed at room temperature. Approximately 600 mL of dichloromethane (CH₂Cl₂) are added. This is extracted with water (3x120 mL).

The decanted dichloromethane is dried with anhydrous magnesium sulphate overnight. On the following day, the MgSO₄ is filtered with a conical funnel and a pleated filter, and the dichloromethane is removed in the rotatory evaporator at atmospheric pressure. When everything has been distilled, it is dried *in vacuo* in the desiccator, yielding a beige-orange solid.

### Synthesis of the anhydride of 2,2,5-trimethyl-[1,3]dioxane-5-carboxylic acid (DMPAA)

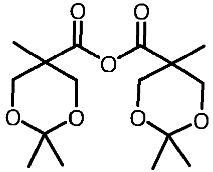

44.6 grams of DMPA (0.256 mol) (174.1g/mol) are dissolved in 179 mL of dichloromethane (CH₂Cl₂) (84.93g/mol); 1.326 g/cm³), (40% w/v) with 26.49 g (0.128 mol) (2:1 stoichiometry) of N,N-dicyclohexylcarbodiimide (DCC) (206.4 g/mol) and allowed to stir for 48 hrs. at room temperature. Lastly, the DDC-urea complex is filtered in vacuo and the solvent is evaporated.

### Synthesis of the 2,2,5-trimethyl-[1,3]dioxane-5-yl)acetic acid 4-metylcoumarin-7-ethoxyl ester

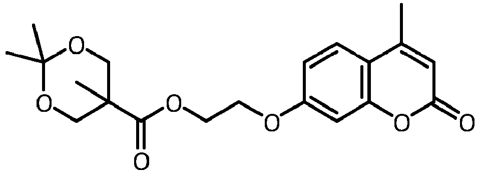

15.1 g of 7-hydroxyethoxy-4-methylcoumarin (0.07 mol) (HEOMC, 220.20 g/mol) (1.5 eq/OH) are added with 4.16g (0.03 mol) of 4-dimethylaminopyridine (DMAP, 122.17 g/mol, 0.2 eq/OH), which are dissolved in 138 mL of anhydrous pyridine (C₅H₅N; 79.10 g/mol; 0.978 g/cm³, 34 eq/OH), and subsequently, are diluted with 554 mL of dichloromethane (CH₂Cl₂; 84.93 g/mol; 1.326 g/cm; 96 eq/OH). Subsequently, the anhydride (DMPAA) 34 g (0,1mol) (330.37g/mol) is added and allowed to react for 9 hours at room temperature. The excess anhydride is destroyed by stirring the reaction overnight with 66 mL of a pyridine:water solution at a ratio of 1:1.

Subsequently, the organic phase is extracted with NaHSO₄ (1M) (120.06 g/mol), subsequently with Na₂CO₃ (105.99 g/mol) at 10%, and lastly with a saturated NaCl solution (58.44 g/mol).

Lastly, the organic phase is dried with anhydrous magnesium sulphate (MgSO₄; 120.37 g/mol) and subsequently filtered and the solvent is evaporated in the rotatory evaporator.

### Synthesis of the 4-hydroxy-3-hydroximethyl-3-methylbutyric acid 4-metylcoumarin-7-ethoxyl ester

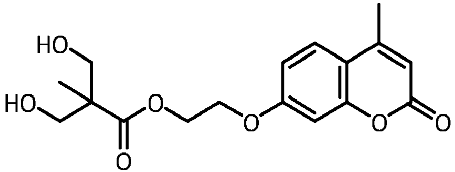

20.9 g (6.021 mmol) of the 2,2,5-trimethyl-[1,3]dioxane-5-yl)acetic acid 4-metylcoumarin-7-ethoxyl ester are dissolved in 200 mL of methanol (MeOH; 32.04 g/mol; 0.792 g/cm³). Subsequently, approximately 10 g of Dowex H⁺ resin are added, filtered and carefully washed with methanol. Methanol is evaporated in the rotatory evaporator to yield the desired compound (white crystals). The obtained product is purified by recrystallization in toluene.

¹H-NMR (400 MHz, Cl₃CD (7.26 ppm) ppm): 7.51 (d, 1H;J=8.8Hz), 6.87 (dd, 1H; J1=8.8Hz, J2=2.4Hz), 6.83 (d, 1H, J=2.4Hz), 6.15 (d, 1H, J=1.2Hz), 4.56 (t, 2H, J=4.8Hz), 4.27 (t, 2H, J=4.8Hz), 3.92 (d, 2H, J=11.2Hz), 3.74 (d, 2H,J=11.2Hz), 2.40 (d, 3H, J=1.2Hz), 1.08 (s, 3H).

### Example 2: Synthesis of the polyol

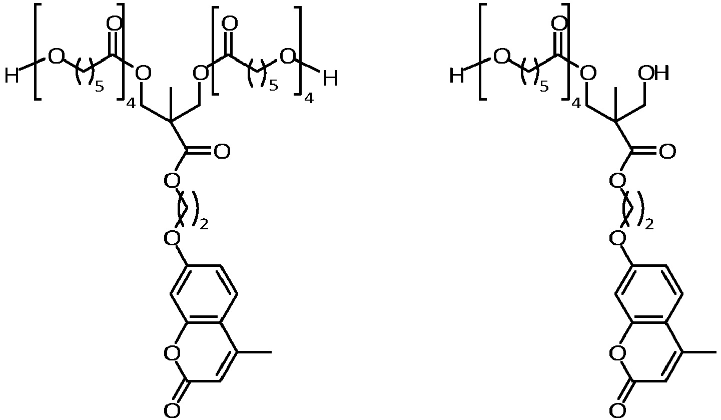

15.5 g (136 mol) of ε-caprolactone and coumarin produced in example 1 (3 g, 8.92 mmol) are added inside the reaction balloon. Then, the tin octanoate (SnOct₂) catalyst is added at a concentration of 0.1% with respect to the weight of the coumarin monomer (compound produced in Example 1). Once all the reagents are added, the reaction is heated to 120° for 24 hrs. under constant stirring. After this period of time, the formed polyol is dried *in vacuo.* The characterization is carried out by ¹H-NMR spectra, determining an average molecular weight thereof of 2054 g/mol. Using this technique, it was possible to verify that a mixture of products had been produced; with a percentage of 66% for the disubstituted polyol and 44% for the monosubstituted polyol.

¹H-NMR (400 MHz, Cl₃CD (7.26 ppm) ppm): 7.51 (d, 1H; J=2.2Hz), 6.83 (d, 2H, J=1.1 Hz), 6.15 (d, 1H, J=1.2Hz), 4.56 (t, 2H, J=4.8Hz), 4.27 (t, 2H, J=4.8Hz), 4.03 (m, 26H), 3.65 (m, 4H), 2.39 (s, 3H), 2.29 (m, 29H), 1.63 (m, 59H), 1.37 (m, 29H) 1.24 (s, 1H), 1.21 (s, 2H).

### Example 3: Synthesis of polyurethane coating, compound of formula (I) as a polyol initiator

1.8 g of the polyol of example 2 is added to a 25 mL balloon with a mixture of linear polyols (0.3 g of butanediol) and polyester polyols (1.2 g of polycaprolactone 527 g/mol) and the corresponding aliphatic isocyanate (1.8 g isophorone diisocyanate). This mixture of components is dissolved in 20 mL of dichloroethane. 2 drops of SnOct₂ catalyst are added and the solution is stirred for 3 hours at 80°C, and subsequently, the product is deposited on a mold for evaporating the solvent.

The mechanical traction properties of the produced product are 0.6 MPa of tensile strength and a modulus of 2 MPa, which are transformed into a tensile strength of 52 MPa and a modulus of 3 MPa after exposure to an ultraviolet light source that causes the dimerization process of the polyol of Example 2.

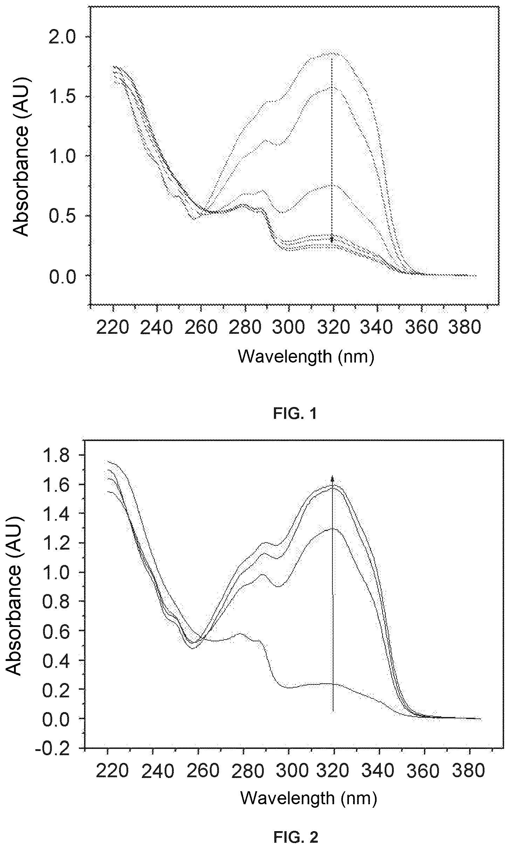

To verify the photoreversibility of the compound, films of 2-5 µm were made for the subsequent characterization thereof using visible UV. The results are shown in Figures 1 and 2. As can be seen in figure 1, when the film is irradiated at a wavelength of 350 nm, the absorbance decreases as a consequence of the dimerization process; that is, the double bond of the coumarin is cleaved, yielding a cyclobutane by the (2+2) cycloaddition reaction. When irradiated at 254 nm, it was observed that the absorbance increased (figure 2) due to the photocleavage process that was taking place in the compound. This is how the initial arrangement of the molecule is recovered.

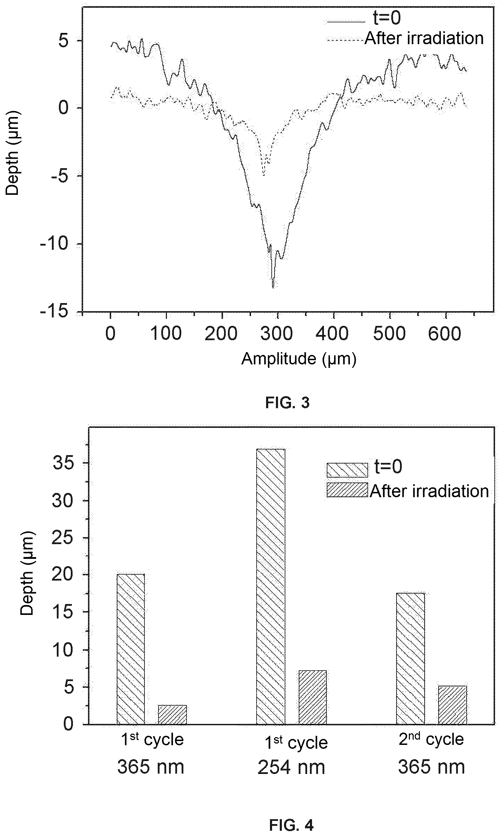

The self-repair was quantified by indentation. By exerting a constant force for a specific period of time, damage was caused on the material. The depth of the damage before and after the application of a UV light source was quantified by confocal microscopy. In this way, the self-repair of the coating was assessed. Figures 3 and 4 show this property.

FTIR-ATR: 3332 u(N-H), 2940 u(CH₂), 2884 u(CH₂), 1725 u(C=O), 1624 u(C=O (urethane groups)), 1551 δ(N-H) u(C-N), 1232 δ(N-H) u(C-N), 1158 u(CO-C).

### Example 4. Synthesis of polyurethane coating, compound of formula I as a chain extender

0.5 g of the coumarin of example 1 is added to a 25 mL balloon. This product is used with a mixture of linear and polyester polyols (0.4 g of butanediol and 2.1 g of polycaprolactone 527 g/mol) and the corresponding aliphatic diisocyanate (2.2 g of isophorone diisocyanate). This mixture of components is dissolved in 20 mL of dichloroethane. 2 drops of SnOct₂ catalyst are added and the solution is stirred for 3 hours at 80°C, and subsequently, the product is deposited on a mold for evaporating the solvent.

The mechanical traction properties of the produced polyurethane are 17 MPa of tensile strength and a modulus of 127 MPa, which are transformed into a tensile strength of 54 MPa and a modulus of 338 MPa after exposure to an ultraviolet light source that causes the dimerization process.

FTIR-ATR: 3350 u(N-H), 2918 u(CH2), 2809 u(CH2), 1703 u(C=O), 1590 u(C=O (urethane groups)), 1511 δ(N-H) u(C-N), 1225 δ(N-H) u(C-N), 1118 u(CO-C).

## Claims

1. A compound of formula (I): 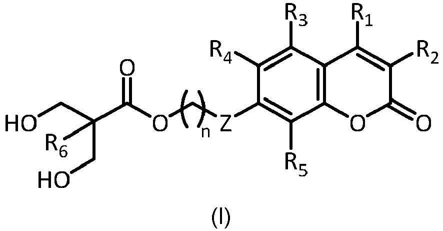
wherein R₁, R₂, R₃, R₄, R₅ and R₆ are independently selected from the group consisting of H, C₁-C₆ alkyl and C₁-C₆ alkoxyl;
Z is selected from the group consisting of CH₂ and O; and n is a number selected from the group consisting of 1, 2, 3 and 4;
or a stereoisomer thereof.

2. The compound of formula (I) according to claim 1, wherein Z is O, and n is selected from the group consisting of 1 and 2.

3. The compound of formula (I) according to any of the previous claims, wherein R₁ and R₆ are independently selected from the group consisting of H, methyl and ethyl, and R₂, R₃, R₄ and R₅ are H.

4. The compound of formula (I) according to any of the previous claims, wherein R₁ and R₆ are methyl, R₂, R₃, R₄ and R₅ are H, Z is O and n is 2.

5. A polyol (A) obtainable by reaction of a compound of formula (I) as defined in any of claims 1 to 4, with a compound (B) in the presence of a catalyst, wherein the compound (B) comprises:
a) a functional group selected from -C(=O)-O- and -O- and optionally a hydroxyl group, provided that when the functional group selected from - C(=O)-O- and -O- does not form part of a cycle, the hydroxyl group must be present; or
b) a -O-CH(CH₃)-(C=O)- functional group and at least one hydroxyl group.

6. The polyol (A) according to claim 5, wherein the compound (B) comprises a functional group selected from -C(=O)-O- and -O- and optionally a hydroxyl group, provided that when the functional group selected from -C(=O)-O- and -O- does not form part of a cycle, the hydroxyl group must be present.

7. The polyol (A) according to any of claims 5 or 6, wherein the compound (B) is a lactone.

8. The polyol (A) according to any of claims 5 to 7, which is a compound of formula (II): 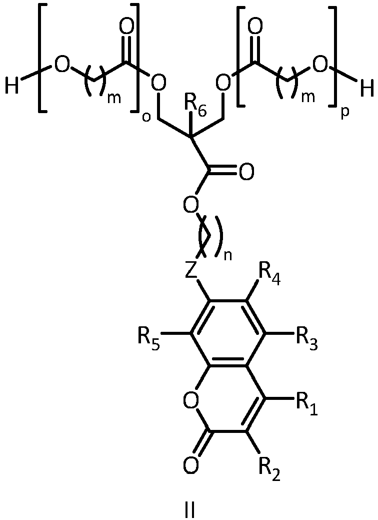 wherein R₁, R₂, R₃, R₄, R₅, R₆, Z and n are as defined in any of claims 1 to 4, and o and p are independently selected from a number comprised between 0 and 40, provided that at least one of o and p is not zero.

9. A method for producing a polyurethane that comprises reacting a mixture comprising:
- one or more polyisocyanates (C) comprising at least two isocyanate groups,
- one or more polyols selected from the group consisting of a polyol (A) as defined in any of claims 5 to 8 and a compound of formula (I) as defined in any of claims 1 to 4 or mixtures thereof, and
- optionally one or more polyols (D) with at least two hydroxyl groups selected from the group consisting of polyester polyols, polyether polyols, aliphatic polyols, polycarbonate polyols, polyether carbonate polyols and polyols of natural origin,
in an aprotic organic solvent,
in the presence of a catalyst,
wherein the ratio of equivalents of polyisocyanate (C) to the sum of equivalents of polyol (A), compound of formula (I) and polyol (D) in the reaction mixture is comprised between 2:1 and 1:1.2, and
wherein the ratio between the sum of the equivalents of polyol (A) and the equivalents of compound of formula (I) with respect to the equivalents of polyisocyanate (C) is comprised between 1% and 95%.

10. A method for producing a polyurethane that comprises reacting a mixture comprising:
- one or more polyisocyanates (C) comprising at least two isocyanate groups,
- one or more polyols (D) with at least two hydroxyl groups selected from the group consisting of polyester polyols, polyether polyols, aliphatic polyols, polycarbonate polyols, polyether carbonate polyols and polyols of natural origin, and
- a compound of formula (I) as defined in any of claims 1 to 4,
in an aprotic organic solvent,
in the presence of a catalyst,
wherein the ratio of equivalents of polyisocyanate (C) to the sum of equivalents of polyol (D) and compound of formula (I) in the reaction mixture is comprised between 2:1 and 1:1.2, and
wherein the ratio between the sum of the equivalents of polyol (D) and the equivalents of compound of formula (I) with respect to the equivalents of polyisocyanate (C) is comprised between 1% and 95%.

11. The method according to any of claims 9 or 10, wherein the polyisocyanate (C) is selected from the group consisting of linear or branched C₂-C₂₀-alkylene diisocyanate optionally substituted with 1 to 10 substituents independently selected from halogens, C₃-C₈-cycloalkylene diisocyanate optionally substituted with 1 to 4 substituents independently selected from the group consisting of C₁-C₃ alkyl and halogen, C₁-C₆-alkylen - C₃-C₈-cycloalkylene diisocyanate optionally substituted with 1 to 4 substituents independently selected from the group consisting of C₁-C₃ alkyl and halogen, and C₃-C₈-cycloalkylen - C₁-C₆-alkylen - C₃-C₈-cycloalkylene diisocyanate optionally substituted with 1 to 4 substituents independently selected from the group consisting of C₁-C₃ alkyl and halogen.

12. The method according to any of claims 9 to 11, wherein the polyol (D) is selected from the group consisting of polycaprolactones, polyethylene glycols, polypropylene glycols, mixed ethylene oxide and propylene oxide polyglycols, polytetramethylene glycols, and polytetrahydrofurans, and the polyol (D) has an average molecular weight between 200 Dalton and 10,000 Dalton.

13. The method according to any of claims 9 to 12, wherein the catalyst is selected from the group consisting of tin octanoate, tin isooctanoate, dibutyltin dilaurate, triethylamine, triethylenediamine, methylethanolamine, triethanolamine, dimethylethanolamine, pyridine, 1,4-diazabicyclo[2.2.2]octane, dimethylcyclohexylamine and potassium octanoate.

14. The method according to any of claims 9 to 13, wherein the aprotic organic solvent is selected from the group consisting of dimethylformamide, butyl acetate and mixtures thereof.

15. The method according to any of claims 9 to 14 comprising:
- reacting a mixture comprising a polyisocyanate (C) selected from the group consisting of hexamethylene diisocyanate, isophorone diisocyanate, tetramethylene diisocyanate, octamethylene diisocyanate, decamethylene diisocyanate, dodecamethylene diisocyanate, and tetradecamethylene diisocyanate,
- a compound of formula (I) as defined in any of claims 1 to 4, and
- a polyol (A) as defined in claim 8, and
- optionally one or more polyols (D) with at least two hydroxyl groups selected from the group consisting of polyester polyols, polyether polyols, aliphatic polyols, polycarbonate polyols, polyether carbonate polyols and polyols of natural origin,
in an aprotic organic solvent,
in the presence of a catalyst,
wherein the ratio of equivalents of polyisocyanate (C) to the equivalents of polyol (A), polyol (D) and compound of formula (I) in the reaction mixture is comprised between 2:1 and 1:1.2, and
wherein the ratio between the sum of the equivalents of polyol (A) and the equivalents of compound of formula (I) with respect to the equivalents of polyisocyanate (C) is comprised between 1% and 95%.

16. A polyurethane obtainable by the method defined in any of claims 9 to 15.

17. Use of a polyurethane according to claim 16 in the preparation of a coating.

18. A method for repairing a coating comprising a polyurethane as defined in claim 17, wherein the method comprises exposing said coating to light comprising a radiation with a wavelength comprised between 310 nm and 370 nm.

19. The method for repairing according to claim 18 which further comprises exposing the damaged polyurethane surface to light comprising a radiation with a wavelength comprised between 240 nm and 260 nm before the exposure as defined in claim 18.
